# EUROPÄISCHE PATENTSCHRIFT

(11) **EP 3 429 640 B1**
(45) Veröffentlichungstag und Bekanntmachung des Hinweises auf die Patenterteilung: **07.05.2025**
(21) Anmeldenummer: 17712943.4
(22) Anmeldetag: 16.03.2017
(51) Int. Cl.: A61L 2/14, A61B 1/00, A61B 17/34, A61B 18/14, H05H 1/24, A61B 18/00

(54) **VORRICHTUNG UND SYSTEM ZUR ANTIMIKROBIELLEN BEHANDLUNG**
DEVICE AND SYSTEM FOR ANTIMICROBIAL TREATMENT
DISPOSITIF ET SYSTÈME DE TRAITEMENT ANTIMICROBIEN

(30) Priorität: 16.03.2016 DE 102016104933
(43) Veröffentlichungstag der Anmeldung: 23.01.2019
(73) Patentinhaber: Leibniz-Institut für Plasmaforschung und Technologie e.V., 17489 Greifswald (DE)
(72) Erfinder: WELTMANN, Klaus-Dieter, 18609 Binz (DE); VON WOEDTKE, Thomas, 18519 Sundhagen (DE); STIEBER, Manfred, 17489 Greifswald (DE); HORN, Stefan, 17509 Loissin (DE)
(74) Vertreter: Schulz Junghans Patentanwälte PartGmbB
(86) Internationale Anmeldenummer: PCT/EP2017/056300
(87) Internationale Veröffentlichungsnummer: WO 2017/158125

(56) Entgegenhaltungen:
- DE-A1- 102014 107 554
- DE-A1- 102014 107 554
- KR-A- 20160 020 082
- KR-A- 20160 020 082
- US-A- 5 156 597
- US-A- 5 156 597
- US-A1- 2013 064 726
- US-A1- 2015 038 790
- US-A1- 2015 202 452
- US-A1- 2015 202 452
- US-B1- 6 432 100
- US-B1- 6 432 100

## Beschreibung

Die Erfindung betrifft eine Vorrichtung und ein System zur antimikrobiellen Behandlung bei der Durchführung von Eingriffen in Körpern.

Für die minimalinvasive Chirurgie werden über chirurgisch angelegte, künstliche Öffnungen in der Körperoberfläche transkutane Zugänge zum Körperinneren gelegt. Zugänge dieser Art sind erfahrungsgemäß durch ein relativ hohes Infektionsrisiko gekennzeichnet, so dass entsprechende infektionsprophylaktische Desinfektions-Maßnahmen erforderlich sind. Eine ähnliche Situation ergibt sich auch für endoskopische Anwendungen in Körperhöhlen.

Da Desinfektionsmittel einerseits allergische Reaktionen verursachen können und andererseits zur Prophylaxe gegen Infektionen wie z. B. mit dem Methicillin-resistenten Staphylococcus aureus (MRSA-Infektionen) nur bedingt geeignet sind, ist die Anwendung einer antimikrobiell wirksamen Plasmabehandlung in diesem Bereich vorteilhaft, zumal bisher keine Resistenzen gegenüber Plasmaeinwirkung bekannt sind. Hierfür ist insbesondere kaltes Atmosphärendruck-Plasma gut geeignet.

Die WO 2015 181 325 A1 bzw. die DE 10 2014 107 554 A1 beschreibt eine Vorrichtung zur biologischen Dekontamination von perkutanen Zugängen oder Stomata mit einem Plasmagenerator. Dieser weist eine gekrümmte Behandlungsfläche auf, die zum Umfassen des perkutanen Zugangs oder des Stomas eingerichtet ist und daran angelegt werden kann. Die Vorrichtung kann beispielsweise als Zange mit beweglichen Zangenbacken ausgeführt sein, an deren Innenseiten Behandlungsflächen angeordnet sind.

Die US 2015/202452 A1 offenbart eine Anwendung von Plasma zur Dekontamination von Kanülen und Endoskopen und schlägt hierzu eine entsprechende Vorrichtung vor. Die Vorrichtungen aus dem Stand der Technik weisen jedoch verschiedene Nachteile auf.

Daher wird ein Gerät zur biologischen Dekontamination (Entkeimung, Desinfektion, Sterilisation) von perkutanen (transkutanen) Zugängen sowie von Hautarealen im Bereich transkutaner Zugänge mittels eines kalten Atmosphärendruckplasmas beschrieben. Die beschriebenen Lösungen zur antimikrobiell wirksamen Plasmabehandlung von perkutanen Zugängen sind zwar vor allem hinsichtlich der Effektivität der biologischen Dekontamination vorteilhaft anzuwenden, erfordern aber den Einsatz einer externen Plasmaquelle als ein zusätzliches Gerät für die Plasmabehandlung.

Es ist die Aufgabe der Erfindung, eine Vorrichtung und ein System zur antimikrobiellen Behandlung bei der Durchführung von Eingriffen in Körpern im Bereich perkutaner Zugänge zur Verfügung zu stellen, mit denen auf besonders einfache und kostengünstige Weise ein Plasma zur antimikrobiellen Behandlung von Körpern erzeugt werden kann.

Die Aufgabe wird gelöst durch die Vorrichtung zur antimikrobiellen Behandlung bei der Durchführung von Eingriffen in Körpern gemäß Anspruch 1 und durch das System zur antimikrobiellen Behandlung bei der Durchführung von Eingriffen in Körpern gemäß Anspruch 9. Ausgestaltungen der Vorrichtung sind in den Unteransprüchen 2-8 angegeben, eine Ausgestaltung des Systems ist in Unteranspruch 10 angegeben.

Ein erster Aspekt der Erfindung ist eine Vorrichtung zur antimikrobiellen Behandlung bei der Durchführung von Eingriffen, insbesondere minimal-invasiven Eingriffen, in Körpern. Diese umfasst einen Grundkörper zum teilweisen Einführen in einen Körper und weiterhin wenigstens eine in zumindest einem Abschnitt des Grundkörpers angeordnete Plasmaquelle. Die Plasmaquelle weist wenigstens eine zumindest teilweise und insbesondere vollständig mit einem Dielektrikum bedeckte Hochspannungselektrode auf, welche dazu eingerichtet ist, bei Anlegen einer elektrischen Spannung und im Zusammenwirken mit einer zweiten Elektrode mittels dielektrisch behinderter Entladung ein Plasma zu erzeugen.

Das Dielektrikum ist im Sinne der Erfindung insbesondere ein Feststoff, der elektrisch schwach bzw. nicht leitet und aus einem nichtmetallischen Werkstoff besteht. Es kann insbesondere ein Kunststoff, beispielsweise Polyethylen oder PTFE, oder ein keramischer Werkstoff sein, wie etwa Steatit, Aluminiumoxid oder Silikat.

Die Hochspannungselektrode ist ein elektrischer Leiter, der zum Anlegen einer Hochspannung geeignet ist. Typischerweise ist sie aus einem metallischen Werkstoff hergestellt. Sie kann dabei ein fester Metallkörper sein oder als Netz, Gewebe oder ähnliches ausgeführt sein, beispielsweise als Metallgaze. Auch eine Ausführung als Draht ist denkbar. Ein solcher kann dabei gewickelt oder mäanderartig geführt sein. Insbesondere ist die Hochspannungselektrode als eine zumindest bereichsweise geschlossene Metallschicht auf den Grundkörper aufgebracht. Sie kann bezogen auf die Längsachse des Grundkörpers in Winkelrichtung umlaufend direkt oder indirekt auf dem Grundkörper angeordnet sein.

Die antimikrobielle Behandlung ist eine desinfizierende, sterilisierende bzw. entkeimende Behandlung. Die Vorrichtung ist demnach z. B. zur biologischen Dekontamination, Entkeimung, Desinfektion bzw. Sterilisation von perkutanen bzw. transkutanen Zugängen sowie von Hautarealen im Bereich derartiger Zugänge geeignet.

Das mit der Vorrichtung erzeugbare Plasma ist insbesondere ein Niedertemperaturplasma, also ein kaltes Atmosphärendruckplasma. Der zugrundeliegende Prozess ist die stille elektrische Entladung bzw. dielektrisch behinderte Entladung. Die Vorrichtung, die zur Durchführung von chirurgischen Eingriffen genutzt werden kann, wird somit selbst zur Plasmaquelle. Der Grundkörper der Vorrichtung ist ein länglicher Grundkörper in Form eines Schaftes eines Trokars oder eines Gerätes für die Endoskopie.

Die zur Erzeugung des Plasmas benötigte zweite Elektrode ist eine geerdete Gegenelektrode, die nicht notwendigerweise Teil der Vorrichtung ist. Sie ist zwecks Erzeugung des Plasmas in einem Abstand zur Hochspannungselektrode anzuordnen, wobei typischerweise zwischen den Elektroden das Dielektrikum anzuordnen ist.

Die elektrische Spannung meint insbesondere eine Wechselspannung. Diese hat typischerweise eine hohe Frequenz im Bereich von 1 kHz bis 1000 kHz. Die elektrische Spannung ist insbesondere eine Hochspannung im Kilovolt-Bereich.

Ein Körper im Sinne der Erfindung ist ein menschlicher oder tierischer Körper oder ein Teil davon.

Wenn der Körper ein menschlicher oder tierischer Körper oder ein Teil davon ist, kann das Einführen durch die Haut (transkutan oder perkutan) oder durch vorhandene Körperöffnungen erfolgen.

Eine Plasmaquelle ist im Wesentlichen durch die erste Hochspannungselektrode ausgebildet, die bei Vorhandensein einer geerdeten Gegenelektrode ein Plasma erzeugen kann, und umfasst hier nicht die Spannungsquelle.

Es wird durch eine elektrisch leitende Beschichtung auf der Oberfläche des Grundkörpers eines minimalinvasiven Gerätes bzw. Endoskopes eine Hochspannungselektrode zur Generierung einer dielektrisch behinderten Entladung (DBE) zwecks Erzeugung eines Plasmas, insbesondere eines Niedertemperaturplasmas, aufgetragen. Dies wird kombiniert mit einer dielektrischen Beschichtung. Dadurch kann das Gerät selbst, nach Anlegen einer geeigneten Hochspannung an die Elektrode, als Plasmaquelle für eine antimikrobiell wirksame Plasmabehandlung im Kontaktbereich des Wundrandes an der Ein- bzw. Austrittsöffnung, genutzt werden, wobei das um- bzw. anliegende Körpergewebe als geerdete Gegenelektrode fungieren kann. Ebenso können Öffnungen aller beliebigen Körper, wie beschrieben, mit Plasma behandelt werden.

Mit anderen Worten wird eine Vorrichtung zur Durchführung eines minimal-invasiven Eingriffs in einen Körper zur Verfügung gestellt, die einen länglichen Grundkörper und wenigstens eine Plasmaquelle umfasst. Die Plasmaquelle ist in zumindest einem Abschnitt des Grundkörpers angeordnet und weist zumindest eine, mit einem Dielektrikum zumindest teilweise und vorzugsweise vollständig bedeckte, Hochspannungselektrode auf, die bei Anlage einer elektrischen Spannung in Zusammenwirkung mit einer zweiten Elektrode ein, auf einer dielektrisch behinderten Entladung basierendes, Plasma erzeugen kann.

Somit wird lokal eine antimikrobielle, Plasma-unterstützte Behandlung im Bereich von transkutanen Zugängen zum Körperinneren oder Zugängen zu Körperhöhlen ermöglicht.

Zweck einer solchen Vorrichtung ist die Inaktivierung von Mikroorganismen an der Eintrittsöffnung und gegebenenfalls im Körperinneren im Umfeld des eingeführten Gerätes, möglicherweise, bei der biologischen Dekontamination von transkutanen Zugängen oder Körperhöhlen an Mensch und Tier, auch die Stimulation der Wundheilung an der Haut-Durchtritts-Öffnung nach Entfernung des Gerätes..

Durch die Erfindung wird eine Minimierung des Infektionsrisikos im Fall minimalinvasiver chirurgischer Eingriffe bzw. endoskopischer Untersuchungen im Bereich der transkutanen Zugänge bzw. Stomata erreicht, wobei ein wesentlicher Vorteil gegenüber den bekannten Lösungen darin besteht dass das Gerät (minimalinvasives Gerät bzw. Endoskop) selbst zur Plasmaerzeugung eingesetzt werden kann und somit keine zusätzliche, externe Plasmaquelle erforderlich ist. Dies wird durch die beschriebene Modifikation der Oberfläche des Gerätegrundkörpers erreicht,

In einer Ausgestaltung der Vorrichtung ist an der der Hochspannungselektrode abgewandten Seite des Dielektrikums in zumindest einem Abschnitt des Grundkörpers ein Abstandselement zur Realisierung eines Abstands zwischen der Plasmaquelle und Körpergewebe angeordnet.

In einer Ausgestaltung ist in einem Abschnitt des Grundkörpers ein Abstandselement zur Realisierung eines Abstands zwischen der Plasmaquelle und Körpergewebe angeordnet.

Das Abstandselement ist insbesondere in Winkelrichtung umlaufend um den Grundkörper angeordnet. Es können dabei mehrere Abschnitte des Grundkörpers ein Abstandselement aufweisen.

Das Abstandselement ist insbesondere nicht elektrisch leitend. Es kann dabei aus einem der beschriebenen Materialien hergestellt sein, aus dem auch das Dielektrikum bestehen kann. Es kann auch zumindest einen Teil des Dielektrikums umfassen oder an diesem anliegen.

Das Abstandselement ist insbesondere als Überzug bzw. Beschichtung außen am Dielektrikum angebracht, so dass es zur Einhaltung eines Mindestabstands zwischen Elektrode bzw. Dielektrikum und jeglichen außen befindlichen Strukturen eingerichtet ist, beispielsweise zu einem beliebigen mit einem Plasma zu behandelnden Körper. Die Dicke der Schicht bzw. die Ausgestaltung der Strukturierung kann dabei in Abhängigkeit der zu behandelnden Oberfläche gewählt werden.

Vorteil dieser Ausgestaltung ist, dass ein definierter Raum zur Plasmaerzeugung zur Verfügung gestellt bzw. ein definierter Abstand zur zu behandelnden Oberfläche gewährleistet wird, der eine Plasmaerzeugung bzw. eine mikrobielle Behandlung unter definierten Bedingungen ermöglicht.

In einer Ausgestaltung ist das Abstandselement aus einem strukturierten, isolierenden, insbesondere elektrisch isolierenden Material hergestellt.

Die Struktur kann dabei z. B. eine strukturierte Oberfläche sein oder durch ein Textil ausgebildet sein, beispielsweise durch ein Gewebe, Gewirk, Gestrick, Geflecht, Nähgewirk, Vliesstoff und/oder ein Filz.

Als Abstandselement kann ein Überzug aus strukturiertem, isolierendem Material (textiles Gewebe, gelochte Silikon-Matte etc.) zur Einhaltung eines definierten Abstandes zwischen dem Körpergewebe und dem als Plasmaquelle genutzten chirurgischen Instrument verwendet werden.

In einer weiteren Ausgestaltung umfasst die Vorrichtung weiterhin die zweite Elektrode, wobei die zweite Elektrode ebenfalls in einem Abschnitt des Grundkörpers angeordnet ist.

Die zweite Elektrode ist, wie beschrieben, eine geerdete Gegenelektrode. Sie kann in Winkelrichtung umlaufend um das Dielektrikum und/oder das Abstandelement angeordnet sein. Auch können mehrere Abschnitte des Grundkörpers die zweite Elektrode bzw. Abschnitte der zweiten Elektrode aufweisen.

In dieser Ausgestaltung ist die Vorrichtung zur Plasmaerzeugung ohne eine externe geerdete Gegenelektrode eingerichtet. Somit kann auch im Bereich nicht leitfähiger Materialien eine antimikrobielle Behandlung erfolgen. Die erfindungsgemäße Vorrichtung kann somit auf besonders vielseitige Weise eingesetzt werden.

In einer weiteren Ausgestaltung ist die zweite Elektrode aus elektrisch leitendem Material, insbesondere aus einem metallischen Gewebe oder einer Metallgaze hergestellt.

Die zweite Elektrode ist somit in einem Abschnitt des Grundkörpers angeordnet, insbesondere in demselben Abschnitt, in dem auch die Hochspannungselektrode angeordnet ist. Sie kann als Textil aus einem metallischen Material ausgestaltet sein, z. B. aus metallischen Fasern oder Faserverbünden. Alle im Zusammenhang mit dem Abstandselement beschriebenen Textilien können dabei einzeln oder in Kombination genutzt werden.

Es kann beispielsweise über dem Abstandselement ein weiterer Überzug aus elektrisch leitendem Material (z.B. Metallgaze) angeordnet sein, der anstelle des Körpergewebes als geerdete Gegenelektrode genutzt wird.

Zusätzlich zu den oben genannten Vorteilen ist hierbei die Effektivität in feuchtem Medium verbessert und es können die Richtlinien der elektromagnetischen Verträglichkeit (EMV) eingehalten werden.

In einer weiteren Ausgestaltung ist die zweite Elektrode an der der Hochspannungselektrode abgewandten Seite des Dielektrikums in zumindest einem Abschnitt des Grundkörpers angeordnet.

Die zweite Elektrode kann außen am Dielektrikum angeordnet sein oder - im Falle des Vorhandenseins eines Abstandselements - außen an diesem.

Bei der erfindungsgemäßen Vorrichtung ist zwischen dem Grundkörper und der Hochspannungselektrode eine hochspannungsfeste Isolationsschicht angeordnet ist, wobei das Dielektrikum die Hochspannungselektrode beidseitig in axialer Richtung überragt, wobei das Dielektrikum in diesen Bereichen die Isolationsschicht kontaktiert und über diese hinaus gehend am Grundkörper anliegt.

Dies ist vorteilhaft, wenn der Grundkörper bzw. dessen äußere Oberfläche aus einem elektrisch leitfähigen Material besteht, so dass der Grundkörper auf diese Weise von der Hochspannungselektrode elektrisch isoliert ist.

Insbesondere ist die Isolationsschicht ebenfalls in Winkelrichtung umlaufend um den Grundkörper angeordnet. Sie kann dabei eine größere Fläche aufweisen als die Hochspannungselektrode und erstreckt sich in axialer Richtung über diese hinaus.

Beispielsweise im Fall eines metallischen Außenrohres des Grundkörpers ist zusätzlich eine hochspannungsfeste Isolationsschicht zwischen dem typischerweise geerdeten Metallrohr und der Metallschicht erforderlich.

Diese Ausführungsform ermöglicht die Nutzung von Gegenständen mit elektrisch leitendem Grundkörper als erfindungsgemäße Vorrichtung.

Erfindungsgemäß ist die Vorrichtung ein Gerät für die Endoskopie oder ein Trokar.

Der Grundkörper der erfindungsgemäßen Vorrichtung ist also der Schaft eines Endoskops oder Trokars. Somit können diese Instrumente gleichzeitig oder alternativ zu ihrer ursprünglichen Funktion als Plasmaquelle eingesetzt werden und vor, während und/oder nach der bekannten Nutzung zur antimikrobiellen Behandlung des Körpers, insbesondere des menschlichen oder tierischen Körpers, genutzt werden. Dabei werden insbesondere die Randbereiche vorhandener oder hergestellter Öffnungen behandelt, um eine Infektion zu verhindern.

Der Schaft bzw. der Grundkörper kann dabei starr oder flexibel sein. Es ist also ebenso möglich, ein flexibles Element wie einen Schlauch mittels geeigneter Ausbildung und Anordnung der Hochspannungselektrode und insbesondere des Dielektrikums derart auszurüsten, dass er als erfindungsgemäße Vorrichtung zur Plasmaerzeugung genutzt werden kann. Starre Grundkörper sind beispielsweise bei Instrumenten für die minimalinvasive Chirurgie wie z. B. Laparoskope vorhanden. Flexible Grundkörper sind beispielsweise Endoskope oder Katheter. Diese können metallische oder nichtmetallische Werkstoffe umfassen.

Der Grundkörper ist erfindungsgemäß ein starres oder flexibles, im Wesentlichen längliches Objekt, das geeignet ist, teilweise in einen Körper eingeführt zu werden. Es kann insbesondere ein Stab, eine Stange, ein Hohlzylinder, ein Seil, eine Seilzugkonstruktion oder ein Schlauch sein.

Bei Ausführung der erfindungsgemäßen Vorrichtung zur Durchführung eines minimal-invasiven Eingriffs als Trokar bietet es sich an, wenn der zur Führung dienende Tubus aus einem elektrisch leitfähigen Material gefertigt ist und als Gegenelektrode dient.

Ein Instrument, vorzugsweise für die minimalinvasive Chirurgie bzw. ein Endoskop, wird demnach erfindungsgemäß zusätzlich als Plasma erzeugendes Gerät genutzt.

Die erfindungsgemäße Vorrichtung kann somit Teil einer medizinischen Einrichtung zur Nutzung in der minimalinvasiven Chirurgie sein. Diese Ausführungsform hat den Vorteil, dass wie beschrieben die bekannte Nutzung durch die antimikrobielle Behandlung ergänzt werden kann. Somit wird auf einfache Weise und ohne zusätzlichen gerätetechnischen Aufwand eine zusätzliche Funktionalität ermöglicht.

Insbesondere weist der Grundkörper der Vorrichtung, ein Verhältnis von Länge zu Durchmesser größer 5:1 und insbesondere größer 10: 1 auf.

Der Durchmesser wird dabei an der dicksten Stelle des Grundkörpers gemessen. Mit anderen Worten ist der Grundkörper länglich. Er kann dabei einen Kreisquerschnitt haben. Insbesondere weist er in einem Abschnitt einen konstanten Querschnitt auf.

Erfindungsgemäß sind die Hochspannungselektrode und das Dielektrikum in zumindest einem Abschnitt des Grundkörpers schichtweise um den gesamten Querschnitt des Grundkörpers herum angeordnet.

Das bedeutet, dass sie als Schichten, die insbesondere jeweils eine konstante Schichtdicke aufweisen können, übereinander liegend um den Grundkörper angeordnet sind.

Insbesondere sind Hochspannungselektrode, Dielektrikum und ggf. weitere Elemente ringförmig und schichtweise um den Grundkörper angeordnet. Somit verlaufen die Schichten jeweils in Winkelrichtung umlaufend um den Grundkörper herum. Diese Ausgestaltung hat den Vorteil, dass die Herstellung der Vorrichtung einfach ist und dass mit dieser im gesamten Winkelbereich um den Grundkörper ein Plasma erzeugt werden kann, so dass alle umlaufend angrenzenden Bereiche des Körpers antimikrobiell behandelt werden können.

Ein zweiter Aspekt ist ein Verfahren zur Herstellung einer erfindungsgemäßen Vorrichtung zur antimikrobiellen Behandlung. Demnach werden die Hochspannungselektrode und das Dielektrikum im Dünnschichtverfahren oder im Dickschichtverfahren auf die Oberfläche des Grundkörpers aufgetragen.

Insbesondere wird zunächst die Hochspannungselektrode und anschließend das Dielektrikum mit je einem der genannten Verfahren auf die Oberfläche aufgetragen. Hier kann jedes Verfahren genutzt werden, mit dem Schichten aus leitfähigem bzw. isolierendem Material aufgebracht werden können. Dabei werden nacheinander Schichten mit den jeweilig gewünschten Eigenschaften aufgetragen bzw. ausgebildet, wobei die jeweilige Schichtdicke von den entsprechenden Prozessparametern abhängt. Dies ermöglicht eine einfache und kostengünstige Herstellung der erfindungsgemäßen Vorrichtung.

Beispielsweise wird durch eine kombinierte metallische und dielektrische Beschichtung im Dünnschicht- oder Dickschichtverfahren auf der Oberfläche des Grundkörpers eines minimalinvasiven Gerätes bzw. Endoskops eine Hochspannungselektrode zur Generierung einer dielektrisch behinderten Entladung (DBE) aufgetragen, wodurch das Gerät selbst, nach Anlegen einer geeigneten Hochspannung an die Elektroden, als Plasmaquelle für eine antimikrobiell wirksame Plasmabehandlung im Kontaktbereich mit dem Körpergewebe, das als geerdete Gegenelektrode fungieren kann, insbesondere im Bereich des Wundrandes an der Ein- bzw. Austrittsöffnung, genutzt werden kann.

Ein dritter Aspekt der Erfindung ist ein System zur antimikrobiellen Behandlung bei der Durchführung von Eingriffen in Körpern. Dieses umfasst eine erfindungsgemäße Vorrichtung zur antimikrobiellen Behandlung sowie eine mit der Vorrichtung elektrisch leitfähig verbundene oder verbindbare Spannungsquelle.

Dabei ist die Spannungsquelle zur Anlegung einer Spannung geeignet, mit welcher mittels der Hochspannungselektrode und einer geerdeten Gegenelektrode durch die Vorrichtung ein Plasma, insbesondere ein Niedrigtemperaturplasma, erzeugt werden kann.

Im Falle eines elektrochirurgischen Gerätes kann die mittel- bzw. hochfrequente Hochspannung, die für die Funktion des Gerätes ohnehin erforderlich ist, während der Behandlung oder beim Herausziehen des Gerätes an die auf dem Grundkörper aufgetragene Elektrode angelegt werden, um die beschriebene Funktion einer Plasmaerzeugung durch das Gerät selbst zu realisieren. In dem Fall wird keine eigene Spannungsquelle benötigt.

Es kann somit eine externe Spannungsquelle oder die verfügbare mittel- bzw. hochfrequente Hochspannungsquelle im Falle eines elektrochirurgischen Gerätes zur Plasmaerzeugung genutzt werden. Ebenfalls kann die verfügbare mittel- bzw. hochfrequente Hochspannungsquelle im Falle eines elektrochirurgischen Gerätes als Spannungsquelle auch zur Plasmaerzeugung mit einer externen Plasmaerzeugungseinrichtung (beispielsweise Plasmaquelle gemäß der WO 2015 181 325 A1) genutzt werden.

Durch den geringeren gerätetechnischen Aufwand wird die Handhabung wesentlich erleichtert und dadurch eine deutliche Zeiteinsparung erreicht.

In einer Ausgestaltung des Systems umfasst dieses den zumindest bereichsweise mit einem Plasma zu behandelnden Körper, wobei zumindest ein Teil des Körpers die zweite Elektrode ausbildet.

Mit anderen Worten wird eine Vorrichtung zur Verfügung gestellt, wobei die zweite Elektrode an oder in dem Körper, in dem der Eingriff durchgeführt werden soll, angeordnet oder durch diesen ausgebildet ist. Somit fungiert der zu untersuchende Körper selbst als geerdete Gegenelektrode. Das System zur Durchführung eines minimal-invasiven Eingriffs weist demnach auch den Körper selbst auf.

Insbesondere umfasst die Vorrichtung selbst in dieser Ausgestaltung nicht die zweite Elektrode.

Ein vierter nicht erfindungsgemäßer Aspekt ist ein Verfahren zur antimikrobiellen Behandlung bei der Durchführung von Eingriffen in Körpern, bei dem die erfindungsgemäße Vorrichtung zur antimikrobiellen Behandlung in den Körper eingeführt wird. Mittels der Vorrichtung wird zumindest in dem Bereich der Öffnung des Körpers, durch den die Vorrichtung zur Durchführung eines minimal-invasiven Eingriffs in den Körpers eingeführt ist, ein Plasma erzeugt. Das Plasma wird zur Durchführung der antimikrobiellen Behandlung typischerweise in Kontakt mit der zu behandelnden Oberfläche gebracht.

Der Körper kann menschliches und/oder tierisches Gewebe umfassen. Unabhängig davon kann das Verfahren am menschlichen oder tierischen Körper oder außerhalb des menschlichen oder tierischen Körpers durchgeführt werden.

Die Erzeugung des Plasmas dient wie beschrieben insbesondere der antimikrobiellen Behandlung des entsprechenden Bereichs des Körpers. Insbesondere wird die Vorrichtung teilweise in den Körper eingeführt, beispielsweise mit ihrem Grundkörper. Dabei kann eine vorhandene Öffnung genutzt werden, beispielsweise im Falle eines Katheters, oder eine Öffnung erzeugt werden, beispielsweise im Falle einer Kanüle oder eines Trokars.

Die Erfindung umfasst weiterhin ein Verfahren zur antimikrobiellen Behandlung bei der Durchführung von Eingriffen in Körpern, nicht zur antimikrobiellen Behandlung menschlichen oder tierischen Gewebes am menschlichen oder tierischen Körper, bei dem die erfindungsgemäße Vorrichtung zur antimikrobiellen Behandlung in den Körper eingeführt wird. Mittels der Vorrichtung wird zumindest in dem Bereich der Öffnung des Körpers, durch den die Vorrichtung zur Durchführung eines minimal-invasiven Eingriffs in den Körpers eingeführt ist, ein Plasma erzeugt. Das Plasma wird zur Durchführung der antimikrobiellen Behandlung typischerweise in Kontakt mit der zu behandelnden Oberfläche gebracht.

Das erfindungsgemäße Verfahren kann mittels Elektronik und Software zur Steuerung einer gleichmäßigen und sicheren Behandlung aktiv überwacht werden.

Ein fünfter Aspekt ist ein Computerprogramm, das alle Schritte zur Durchführung des Verfahrens zur antimikrobiellen Behandlung ausführt, wenn das Programm auf einem Computer läuft.

Das Computerprogramm kann dabei die Plasmaerzeugung steuern: Beispielsweise kann es diese starten, beenden, ein definiertes Programm abfahren und/oder überwachen. Es kann weiterhin zur Verarbeitung und Nutzung von Messwerten eingerichtet sein.

Insbesondere kann dies ein Computerprogramm sein, das alle Schritte zur Durchführung eines Verfahrens zur antimikrobiellen Behandlung bei der Durchführung von Eingriffen in Körpern ausführt, wenn das Programm auf einem Computer läuft. Bei dem genannten Verfahren wird die erfindungsgemäße Vorrichtung zur antimikrobiellen Behandlung in den Körper eingeführt und mittels der Vorrichtung wird zumindest in dem Bereich der Öffnung des Körpers, durch den die Vorrichtung zur Durchführung eines minimal-invasiven Eingriffs in den Körpers eingeführt ist, ein Plasma erzeugt. Alternativ kann das Computerprogramm eines sein, das alle Schritte zur Durchführung eines Verfahrens zur antimikrobiellen Behandlung bei der Durchführung von Eingriffen in Körpern, nicht zur antimikrobiellen Behandlung menschlichen oder tierischen Gewebes am menschlichen oder tierischen Körper, ausführt, wenn das Programm auf einem Computer läuft. Bei dem genannten Verfahren wird die erfindungsgemäße Vorrichtung zur antimikrobiellen Behandlung in den Körper eingeführt wird und mittels der Vorrichtung wird zumindest in dem Bereich der Öffnung des Körpers, durch den die Vorrichtung zur Durchführung eines minimal-invasiven Eingriffs in den Körpers eingeführt ist, ein Plasma erzeugt.

Ein weiterer Aspekt ist ein Computerprogramm-Produkt mit einem auf einem maschinenlesbaren Träger gespeicherten Programmcode zur Durchführung des Verfahrens, wenn das Programm von einem Computer, insbesondere von einem, in einem elektronischen Steuergerät integrierten, Mikrokontroller, ausgeführt wird.

Ebenso Gegenstand der Erfindung ist ein Computerprogramm-Produkt, mit einem auf einem maschinenlesbaren Träger gespeicherten Programmcode des erfindungsgemäßen Computerprogramms. Maschinenlesbare Träger können alle Datenträger, insbesondere alle digitalen Datenträger, sein.

Die Erfindung wird im Folgenden anhand des in den beiliegenden Zeichnungen dargestellten Ausführungsbeispiels erläutert.

Es zeigen
Fig. 1: eine perspektivische Darstellung einer erfindungsgemäßen Vorrichtung, ausgeführt als Laparoskop, sowie
Fig. 2: eine geschnittene Darstellung eines Details aus dem Grundkörper der Vorrichtung aus Fig. 1.

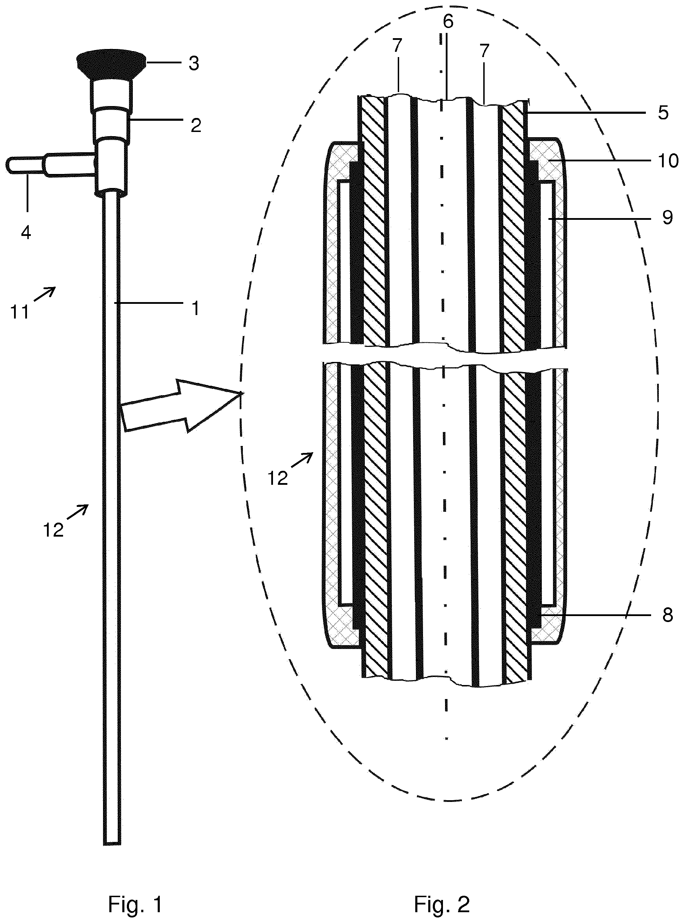

Figur 1 zeigt die erfindungsgemäße Vorrichtung 11, ausgeführt als Laparoskop. Dieses ist ein einfaches Gerät für die minimalinvasive Chirurgie und umfasst einen länglichen Grundkörper 1, der zum wenigstens teilweisen Einführen in einen Körper, insbesondere einen menschlichen oder tierischen Körper, eingerichtet ist. An der oben gezeigten Seite des Grundkörpers, die der in den Körper einzuführenden Seite gegenüberliegt, ist ein Handstück 2 mit einem Okular 3 und einem seitlichen Lichtleiteranschluss 4 angeordnet.

An den Lichtleiteranschluss 4 schließt sich im Inneren des Grundkörpers 1 ein Lichtkanal 7 an, in dem Licht von einer externen, an den Lichtleiteranschluss 4 anzuschließenden Lichtquelle ins Innere des zu untersuchenden Körpers geleitet werden kann. Der Lichtkanal 7 ist kreisringförmig ausgeführt und umschließt den darin liegenden Optikkanal 6, der zur Leitung der von Oberflächen im Körperinneren reflektierten Lichtstrahlung hin zum Okular 3 eingerichtet ist. Somit können optische Informationen aus dem Körperinneren an den Anwender der Vorrichtung geleitet werden. Die genannten optisch leitenden Kanäle 6, 7 sind in der in Figur 2 gezeigten Schnittzeichnung sichtbar.

Der Grundkörper 1 ist als Metallrohr mit Kreisquerschnitt ausgeführt, im dem Einbauten zwecks Unterteilung der genannten optisch leitenden Kanäle 6, 7 angeordnet sind.

Erfindungsgemäß ist das Außenrohr 5 des Grundkörpers 1 in einem Teilbereich der Länge rundum mehrlagig in einer definierten Weise beschichtet, um diesen Bereich als Hochspannungselektrode 9 zur Erzeugung eines Plasmas nutzen zu können. Um den dafür erforderlichen prinzipiellen Schichtaufbau zu demonstrieren, ist Figur 2 ein Teil des beschichteten Grundkörpers 1 in einer vergrößerten Schnittzeichnung dargestellt.

Auf dem metallischen Außenrohr 5 des Grundkörpers 1 ist eine hochspannungsfeste Isolationsschicht 8 angeordnet, die den Grundkörper 1, der geerdet ausgeführt ist, elektrisch von der um diesen herum angeordneten Hochspannungselektrode 9 isoliert. Somit wird zwischen Grundkörper 1 und Hochspannungselektrode 9 eine elektrische Potentialdifferenz ermöglicht, die zur Erzeugung des Plasmas notwendig ist.

Die Hochspannungselektrode 9 zur Erzeugung einer dielektrisch behinderten Entladung wird durch eine mit einer Dielektrikum-Schicht 10 bedeckten Metallschicht gebildet. Das Dielektrikum 10 überragt dabei die Hochspannungselektrode 9 beidseitig in axialer Richtung, erstreckt sich also über das obere und unter das untere Ende der Hochspannungselektrode 9 hinaus, wobei es in diesen Bereichen die Isolationsschicht 8 kontaktiert und über diese hinaus gehend am metallischen Grundkörper 1 anliegt. Somit dient das Dielektrikum 10 als Ummantelung bzw. Überzug, der die beschriebenen Schichten beispielsweise vor Feuchtigkeit schützt.

Die beschriebenen Schichten sind über einen Großteil des in den Körper einführbaren Bereichs des Grundkörpers 1 angeordnet.

Je nach Beschaffenheit der Grundkörper 1 der minimalinvasiven Geräte oder Endoskope (Oberfläche, Form, Größe und Materialart) kann die Erfindung auf unterschiedliche Weisen technisch umgesetzt werden, so dass daraus verschiedene Varianten der Realisierung resultieren , ohne vom Schutzbereich des Anspruchs 1 abzuweichen.

Für die Plasmaerzeugung ist dabei lediglich eine externe Stromversorgung erforderlich.

### Bezugszeichenliste

- 1: Grundkörper
- 2: Handstück
- 3: Okular
- 4: Lichtleiteranschluss
- 5: Außenrohr des Grundkörpers
- 6: Optikkanal
- 7: Lichtkanal
- 8: Isolationsschicht
- 9: Hochspannungselektrode (Metallschicht)
- 10: Dielektrikum (Dielektrikum-Schicht)
- 11: Vorrichtung
- 12: Plasmaquelle

## Patentansprüche

1. Vorrichtung (11) zur antimikrobiellen Behandlung im Bereich perkutaner Zugänge bei der Durchführung von Eingriffen, insbesondere minimal-invasiven Eingriffen, in Körpern, mit einem Grundkörper (1), in Form eines Schafts zum teilweisen Einführen in einen Körper, wobei die Vorrichtung (11) ein Gerät für die Endoskopie oder ein Trokar ist,
wobei die Vorrichtung (11) weiterhin wenigstens eine in zumindest einem Abschnitt des Grundkörpers (1) angeordnete Plasmaquelle (12) umfasst, wobei die Plasmaquelle (12) wenigstens eine zumindest teilweise und insbesondere vollständig mit einem Dielektrikum (10) bedeckte Hochspannungselektrode (9) aufweist, welche dazu eingerichtet ist, bei Anlegen einer elektrischen Spannung und im Zusammenwirken mit einer zweiten Elektrode mittels dielektrisch behinderter Entladung ein Plasma zu erzeugen, **dadurch gekennzeichnet, dass** die Hochspannungselektrode (9) und das Dielektrikum (10) in zumindest einem Abschnitt des Grundkörpers (1) schichtweise um einen Querschnitt des Grundkörpers (1) herum angeordnet sind, wobei zwischen dem Grundkörper (1) und der Hochspannungselektrode (9) eine hochspannungsfeste Isolationsschicht (8) angeordnet ist, wobei das Dielektrikum (10) die Hochspannungselektrode (9) beidseitig in axialer Richtung überragt, wobei das Dielektrikum (10) in diesen Bereichen die Isolationsschicht (8) kontaktiert und über diese hinaus gehend am Grundkörper (1) anliegt.

2. Vorrichtung (11) zur antimikrobiellen Behandlung nach Anspruch 1, **dadurch**
**gekennzeichnet, dass** an der der Hochspannungselektrode (9) abgewandten Seite des Dielektrikums (10) in zumindest einem Abschnitt des Grundkörpers (1) ein Abstandselement zur Realisierung eines Abstands zwischen der Plasmaquelle (12) und Körpergewebe angeordnet ist.

3. Vorrichtung (11) zur antimikrobiellen Behandlung nach Anspruch 2, **dadurch**
**gekennzeichnet, dass** das Abstandselement aus einem strukturierten, isolierenden, insbesondere elektrisch isolierenden Material hergestellt ist.

4. Vorrichtung (11) zur antimikrobiellen Behandlung nach einem der vorhergehenden Ansprüche, **dadurch gekennzeichnet, dass** diese weiterhin die zweite Elektrode umfasst, wobei die zweite Elektrode ebenfalls in einem Abschnitt des Grundkörpers (1) angeordnet ist.

5. Vorrichtung (11) zur antimikrobiellen Behandlung nach Anspruch 4, **dadurch gekennzeichnet, dass** die zweite Elektrode aus elektrisch leitendem Material, insbesondere einem metallischen Gewebe oder einer Metallgaze hergestellt ist.

6. Vorrichtung (11) zur antimikrobiellen Behandlung nach einem der Ansprüche 4 und 5, **dadurch gekennzeichnet, dass** die zweite Elektrode an der der Hochspannungselektrode (9) abgewandten Seite des Dielektrikums (10) in zumindest einem Abschnitt des Grundkörpers (1) angeordnet ist.

7. Vorrichtung (11) zur antimikrobiellen Behandlung nach einem der vorhergehenden Ansprüche, **dadurch gekennzeichnet, dass** die Vorrichtung (11) ein Gerät für die Laparoskopie ist.

8. Vorrichtung (11) zur antimikrobiellen Behandlung nach einem der vorhergehenden Ansprüche, **dadurch gekennzeichnet, dass** das Abstandselement, die zweite Elektrode und/oder das elektrisch isolierende Element, in zumindest einem Abschnitt des Grundkörpers (1) schichtweise um den gesamten Querschnitt des Grundkörpers (1) herum angeordnet sind.

9. System zur antimikrobiellen Behandlung bei der Durchführung von Eingriffen in Körpern, **dadurch gekennzeichnet, dass** das System eine Vorrichtung (11) zur antimikrobiellen Behandlung gemäß einem der Ansprüche 1 bis 8 sowie eine mit der Vorrichtung (11) elektrisch leitfähig verbundene oder verbindbare Spannungsquelle umfasst.

10. System zur antimikrobiellen Behandlung nach Anspruch 9, **dadurch**
**gekennzeichnet, dass** das System den zumindest bereichsweise mit einem Plasma zu behandelnden Körper umfasst, wobei zumindest ein Teil des Körpers die zweite Elektrode ausbildet.

## Claims

1. A device (11) for antimicrobial treatment in the region of percutaneous accesses when performing interventions, in particular minimally invasive interventions, in bodies, having a base body (1) in the form of a shaft for partial insertion into a body, the device (11) being a device for endoscopy or a trocar,
wherein the device (11) further comprises at least one plasma source (12) arranged in at least one section of the basic body (1), wherein the plasma source (12) has at least one high-voltage electrode (9) which is at least partially and in particular completely covered with a dielectric (10) and which is configured to generate a plasma when an electrical voltage is applied and in cooperation with a second electrode by means of a dielectric barrier discharge, **characterized in that** in at least one section of the base body (1) the high-voltage electrode (9) and the dielectric (10) are arranged in layers around a cross-section of the base body (1), wherein a high-voltage-resistant insulating layer (8) is arranged between the base body (1) and the high-voltage electrode (9), wherein the dielectric (10) projects beyond the high-voltage electrode (9) on both sides in the axial direction, wherein the dielectric (10) contacts the insulating layer (8) in these portions and lies against the base body (1) beyond these portions.

2. The device (11) for antimicrobial treatment according to claim 1, **characterized in that** a spacer element is arranged on the side of the dielectric (10) facing away from the high-voltage electrode (9) in at least one section of the base body (1) in order to create a distance between the plasma source (12) and body tissue.

3. The device (11) for antimicrobial treatment according to claim 2, **characterized in that** the spacer element is made of a structured, insulating, in particular electrically insulating material.

4. The device (11) for antimicrobial treatment according to one of the preceding claims, **characterized in that** it further comprises the second electrode, wherein the second electrode is also arranged in a section of the base body (1).

5. The device (11) for antimicrobial treatment according to claim 4, **characterized in that** the second electrode is made of electrically conductive material, in particular a metallic fabric or a metallic gauze.

6. The device (11) for antimicrobial treatment according to one of claims 4 and 5, **characterized in that** the second electrode is arranged on the side of the dielectric (10) facing away from the high-voltage electrode (9) in at least one section of the base body (1).

7. The device (11) for antimicrobial treatment according to one of the preceding claims, **characterized in that** the device (11) is an apparatus for laparoscopy.

8. The device (11) for antimicrobial treatment according to one of the preceding claims,
**characterized in that** the spacer element, the second electrode and/or the electrically insulating element are arranged in layers around the entire cross-section of the base body (1) in at least one section of the base body (1).

9. A system for antimicrobial treatment when performing interventions in bodies, **characterized in that** the system comprises a device (11) for antimicrobial treatment according to one of claims 1 to 8 and a voltage source electrically conductively connected or connectable to the device (11).

10. The system for antimicrobial treatment according to claim 9, **characterized in that** the system comprises the body to be treated with a plasma at least in some area, wherein at least a part of the body forms the second electrode.

## Revendications

1. Dispositif (11) de traitement antimicrobien dans la région d'accès percutanés lors de la réalisation d'interventions, notamment d'interventions minimalement invasives, dans des corps, avec un corps de base (1), sous forme d'une tige pour l'introduction partielle dans un corps, dans lequel le dispositif (11) est un appareil pour l'endoscopie ou un trocart,
dans lequel le dispositif (11) comprend en outre au moins une source de plasma (12) disposée dans au moins une section du corps de base (1), dans lequel la source de plasma (12) présente au moins une électrode haute tension (9) recouverte au moins partiellement et notamment entièrement d'un diélectrique (10) qui est configurée pour générer un plasma lors de l'application d'une tension électrique et en coopération avec une seconde électrode au moyen de décharge diélectriquement empêchée, **caractérisé en ce que** l'électrode haute tension (9) et le diélectrique (10) sont disposés dans au moins une section du corps de base (1) par couches autour d'une section transversale du corps de base (1), dans lequel une couche isolante (8) résistant aux hautes tensions est disposée entre le corps de base (1) et l'électrode haute tension (9), dans lequel le diélectrique (10) dépasse de l'électrode haute tension (9) des deux côtés en direction axiale, dans lequel le diélectrique (10) vient en contact avec la couche isolante (8) dans ces régions et repose sur le corps de base (1) en le dépassant.

2. Dispositif (11) de traitement antimicrobien selon la revendication 1, **caractérisé en ce qu'**un écarteur est disposé sur le côté du diélectrique (10) détourné de l'électrode haute tension (9) dans au moins une section du corps de base (1) pour la réalisation d'un écart entre la source de plasma (12) et du tissu corporel.

3. Dispositif (11) de traitement antimicrobien selon la revendication 2, **caractérisé en ce que** l'écarteur est fabriqué en un matériau structuré, isolant, notamment électriquement isolant.

4. Dispositif (11) de traitement antimicrobien selon une des revendications précédentes, **caractérisé en ce que** celui-ci comprend en outre la seconde électrode, dans lequel la seconde électrode est également disposée dans une section du corps de base (1).

5. Dispositif (11) de traitement antimicrobien selon la revendication 4, **caractérisé en ce que** la seconde électrode est fabriquée en matériau électriquement conducteur, notamment un tissu métallique ou une gaze métallique.

6. Dispositif (11) de traitement antimicrobien selon une des revendications 4 et 5,
**caractérisé en ce que** la seconde électrode est disposée sur le côté du diélectrique (10) détourné de l'électrode haute tension (9) dans au moins une section du corps de base (1).

7. Dispositif (11) de traitement antimicrobien selon une des revendications précédentes, **caractérisé en ce que** le dispositif (11) est un appareil pour la laparoscopie.

8. Dispositif (11) de traitement antimicrobien selon une des revendications précédentes, **caractérisé en ce que** l'écarteur, la seconde électrode et/ou l'élément électriquement isolant sont disposés dans au moins une section du corps de base (1) par couches autour de la section transversale entière du corps de base (1).

9. Système de traitement antimicrobien lors de la réalisation d'interventions dans des corps, **caractérisé en ce que** le système comprend un dispositif (11) de traitement antimicrobien selon une des revendications 1 à 8 ainsi qu'une source de tension connectée ou pouvant être connectée de manière électriquement conductrice au dispositif (11).

10. Système de traitement antimicrobien selon la revendication 9, **caractérisé en ce que** le système comprend le corps à traiter avec un plasma au moins par régions, dans lequel au moins une partie du corps forme la seconde électrode.
